# EUROPÄISCHE PATENTSCHRIFT

(11) **EP 3 197 520 B1**
(45) Veröffentlichungstag und Bekanntmachung des Hinweises auf die Patenterteilung: **16.12.2020**
(21) Anmeldenummer: 15767147.0
(22) Anmeldetag: 22.09.2015
(51) Int. Cl.: A61M 1/36

(54) **VORRICHTUNGEN ZUM VORBEREITEN EINES EXTRAKORPORALEN BLUTKREISLAUFS FÜR DIE BLUTBEHANDLUNG**
DEVICES FOR PREPARING AN EXTRACORPOREAL BLOOD CIRCUIT FOR THE BLOOD TREATMENT
DISPOSITIFS POUR LA PRÉPARATION D'UNE CIRCULATION SANGUINE EXTRACORPORELLE POUR LE TRAITEMENT DU SANG

(30) Priorität: 23.09.2014 DE 102014113728
(43) Veröffentlichungstag der Anmeldung: 02.08.2017
(73) Patentinhaber: Fresenius Medical Care Deutschland GmbH, 61352 Bad Homburg (DE)
(72) Erfinder: POHLMEIER, Robert, 61350 Bad Homburg (DE); WEHMEYER, Wolfgang, 72076 Tübingen (DE)
(74) Vertreter: Bobbert & Partner Patentanwälte PartmbB
(86) Internationale Anmeldenummer: PCT/EP2015/071677
(87) Internationale Veröffentlichungsnummer: WO 2016/046177

(56) Entgegenhaltungen:
- WO-A2-01/37900
- CA-A1- 2 863 264

## Beschreibung

Die vorliegende Erfindung betrifft eine Steuer- oder Regelungseinrichtung gemäß Anspruch 1 zum Vorbereiten eines extrakorporalen Blutkreislaufs für die Blutbehandlung. Zudem betrifft sie eine Blutbehandlungsvorrichtung gemäß Anspruch 11. In der Praxis der extrakorporalen Blutbehandlung unter Einsatz eines extrakorporalen Blutkreislaufs ist es erforderlich und üblich, den extrakorporalen Blutkreislauf vor Aufnahme der Blutbehandlung zu primen. Hierunter wird hierin ein Befüllen des nach seiner Fertigung mit Luft gefüllten extrakorporalen Blutkreislaufs mittels eines flüssigen Fluids verstanden, was das spätere Eintragen von Luft in das Gefäßsystem des Patienten verhindern soll. Die zum Primen verwendete Lösung wird hierin als Priminglösung verstanden. Unter einer Priminglösung kann eine Mischung aus Lösungen verstanden werden, welche gemeinsam zum Priming verwendet werden. Das Dokument CA2863264 A1 beschreibt eine Blutbehandlung mit Zitratantikoagulation.

Eine Aufgabe der vorliegenden Offenbarung ist es, ein Verfahren und Vorrichtungen zum Vorbereiten des extrakorporalen Blutkreislaufs anzugeben. Ferner sollen eine Blutbehandlungsvorrichtung, mit welcher das offenbarte, jedoch nicht beanspruchte Verfahren durchführbar ist, sowie eine zur Durchführung des Verfahrens vorgesehene Steuereinrichtung, ein geeignetes digitales Speichermedium, ein geeignetes Computerprogramm-Produkt und ein geeignetes Computerprogramm angegeben werden.

Die erfindungsgemäße Aufgabe wird durch eine Steuereinrichtung mit den Merkmalen des Anspruchs 1 sowie eine Blutbehandlungsvorrichtung mit den Merkmalen des Anspruchs 11 gelöst. Ferner werden ein digitales Speichermedium, ein Computerprogramm-Produkt sowie ein Computerprogramm hierin beschrieben, aber nicht beansprucht.

Alle mit dem offenbarten, nicht-erfindungsgemäßen Verfahren erzielbaren Vorteile lassen sich in auch mit den erfindungsgemäßen Vorrichtungen erzielen.

Das offenbarte, nicht-erfindungsgemäße Verfahren betrifft das Vorbereiten eines extrakorporalen Blutkreislaufs für seine Verwendung bei der Blutbehandlung eines Patienten, zu welcher eine Blutbehandlungsvorrichtung verwendet wird. Das Verfahren kann ein Bereitstellen einer geeigneten Blutbehandlungsvorrichtung umfassen.

Die geeignete Blutbehandlungsvorrichtung weist wenigstens eine Blutbehandlungseinrichtung, beispielsweise einen Blutfilter oder einen Dialysator, auf. Die Blutbehandlungseinrichtung weist vorzugsweise eine Blutkammer und eine mittels Membran hiervon abgeteilte Dialysierflüssigkeitskammer auf.

Die Blutbehandlungsvorrichtung weist zum Zweck der Blutbehandlung des Patienten die im Folgenden genannten Einrichtungen und/oder Vorrichtungen auf oder ist mit ihnen in Fluid- und/oder Signalverbindung verbindbar oder verbunden.

Das offenbarte, nicht-erfindungsgemäße Verfahren umfasst ein Befüllen des extrakorporalen Blutkreislaufs oder vorbestimmter Abschnitte hiervon mittels einer Priminglösung. Dies geschieht vor Beginn der Blutbehandlung. Die Priminglösung weist Zitrat auf. Alternativ oder ergänzend umfasst das Verfahren ein Befüllen des extrakorporalen Blutkreislaufs oder vorbestimmter Abschnitte hiervon mittels einer Priminglösung und mittels einer, beispielsweise konzentrierten oder hochkonzentrierten, Zitratlösung. Auch die Mischung aus Priminglösung, etwa in Form einer Dialysierflüssigkeit oder eines Substituats, und einer Zitratlösung, wird hierin vereinfacht als Priminglösung bezeichnet, sofern zum Primen verwendet.

Die erfindungsgemäße Steuer- oder Regelungseinrichtung ist programmiert zur Durchführung wenigstens einer Ausführungsform des Verfahrens, im Zusammenwirken mit den hierzu jeweils erforderlichen Einrichtungen, beispielsweise wie hierin beschrieben.

Die erfindungsgemäße Blutbehandlungsvorrichtung weist eine erfindungsgemäße Steuer- oder Regeleinrichtung auf, welche programmiert ist, um das offenbarte, nicht-erfindungsgemäße Verfahren im Zusammenwirken mit weiteren Einrichtungen, insbesondere einer Blutbehandlungsvorrichtung, auszuführen. Die erfindungsgemäße Blutbehandlungsvorrichtung ist vorgesehen und/oder ausgestaltet und/oder ausgestattet zur Durchführung des offenbarten, nicht-erfindungsgemäßen Verfahrens.

Ein beispielhaftes digitales, insbesondere nicht-flüchtiges, Speichermedium, insbesondere in Form eines maschinenlesbaren Trägers, insbesondere in Form einer Diskette, CD, EPROM oder DVD, mit elektrisch auslesbaren Steuersignalen kann derart mit einem programmierbaren Computersystem zusammenwirken, dass die maschinellen Schritte eines offenbarten, nicht-erfindungsgemäßen Verfahrens veranlasst werden.

Dabei können alle, einige oder manche der maschinell durchgeführten Schritte des Verfahrens veranlasst werden.

Ein beispielhaftes Computerprogramm-Produkt weist einen volatilen, flüchtigen oder auf einem maschinenlesbaren Träger gespeicherten Programmcode oder eine Signalwelle zur Veranlassung der maschinellen Schritte des offenbarten, nicht-erfindungsgemäßen Verfahrens, wenn das Computerprogramm-Produkt auf einem Rechner abläuft, auf. Unter einem Computerprogramm-Produkt kann beispielsweise ein auf einem Träger gespeichertes Computerprogramm, ein Embedded System als umfassendes System mit einem Computerprogramm (z. B. elektronisches Gerät mit einem Computerprogramm), ein Netzwerk von computerimplementierten Computerprogrammen (z. B. Client/Serversystem, Cloud Computing System, etc.), oder ein Computer, auf dem ein Computerprogramm geladen ist, abläuft, gespeichert ist, ausgeführt oder entwickelt wird, verstanden werden.

Der Begriff "maschinenlesbarer Träger", wie er hierin verwendet wird, bezeichnet in bestimmten Ausführungsformen der vorliegenden : Offenbarung einen Träger, der von Software und/oder Hardware interpretierbare Daten oder Informationen enthält. Der Träger kann ein Datenträger, wie eine Diskette, eine CD, DVD, ein USB-Stick, eine Flashcard, eine SD-Karte oder dergleichen sein.

Ein beispielhaftes Computerprogramm weist einen Programmcode auf zur Veranlassung der maschinellen Schritte eines offenbarten, nicht-erfindungsgemäßen Verfahrens, wenn das Computerprogramm auf einem Computer abläuft. Unter einem Computerprogramm kann beispielsweise ein physikalisches, vertriebsfähiges Software-Produkt verstanden werden, welches ein Programm aufweist.

Auch für das beispielhafte Computerprogramm-Produkt und das beispielhafte Computerprogramm gilt, dass alle, einige oder manche der maschinell durchgeführten Schritte des offenbarten, nicht-erfindungsgemäßen Verfahrens veranlasst werden.

Erfindungsgemäße Ausführungsformen können manche, einige oder alle der folgenden Merkmale in beliebiger Kombination aufweisen, soweit dies für den Fachmann nicht erkennbar technisch unmöglich ist. Die Erfindung betrifft aber eine Steuer- oder Regelungseinrichtung gemäß Anspruch 1. Vorteilhafte Weiterbildungen der vorliegenden Erfindung sind jeweils auch Gegenstand der Unteransprüche.

Bei allen folgenden Ausführungen ist der Gebrauch des Ausdrucks "kann sein" bzw. "kann haben" usw. synonym zu "ist vorzugsweise" bzw. "hat vorzugsweise" usw. zu verstehen und soll erfindungsgemäße Ausführungsformen erläutern.

Wann immer hierin Zahlenworte genannt werden, so versteht der Fachmann diese als Angabe einer zahlenmäßig unteren Grenze.

Sofern dies zu keinem für den Fachmann erkennbaren Widerspruch führt, liest der Fachmann daher beispielsweise bei der Angabe "ein" oder "einem" stets "wenigstens ein" oder "wenigstens einem" mit. Dieses Verständnis ist ebenso von der vorliegenden Erfindung mit umfasst wie die Auslegung, dass ein Zahlenwort wie beispielsweise "ein" alternativ als "genau ein" gemeint sein kann, wo immer dies für den Fachmann erkennbar technisch möglich ist. Beides ist von der vorliegenden Erfindung umfasst und gilt für alle hierin verwendeten Zahlenworte.

In einigen Ausführungsformen unterscheidet sich die Priminglösung von Blut oder ist optional gar vollkommen frei von Blut.

In bestimmten Ausführungsformen erfolgt das Befüllen derart, dass die Priminglösung, mit welcher der extrakorporale Blutkreislauf befüllt ist, maximal 0,5 mmol/L oder maximal 0,6 mmol/L, insbesondere ionisierten, Kalziums aufweist, insbesondere vor Beginn der Blutbehandlung.

In einigen Ausführungsformen wird die - beispielsweise konzentrierte oder hochkonzentrierte - Zitratlösung der Priminglösung zugegeben, bevor mittels dieser der extrakorporale Blutkreislauf befüllt wird.

In bestimmten Ausführungsformen erfolgt das Befüllen mit einer Priminglösung mit einem pH-Wert von wenigstens 7,3, insbesondere messbar vor Beginn der Blutbehandlung.

In einigen Ausführungsformen weist die Priminglösung die übliche physiologische Zusammensetzung auf, insbesondere vor Beginn der Blutbehandlung.

In bestimmten Ausführungsformen umfasst das Verfahren weiter das Befüllen der Dialysierflüssigkeitskammer der Blutbehandlungseinrichtung über die Membran hinweg oder durch diese hindurch. Dabei wandert ein Teil der hierin beschriebenen Priminglösung (welche, wie oben ausgeführt, eine Zitratlösung umfassen kann) von der Blutkammer der Blutreinigungseinrichtung, z. B. des Blutfilters, auf die Seite der Dialysierflüssigkeitskammer und füllt diese mehr oder weniger.

Alternativ oder ergänzend kann die Befüllung der Dialysierflüssigkeitskammer über die Dialyierflüssigkeitsseite erfolgen, und zwar mit einer Lösung, welche dieselben Eigenschaften wie die Priminglösung, jedenfalls hinsichtlich der Kalziumkonzentration und/oder der Zitatkonzentration hat.

In einigen Ausführungsformen umfasst das Verfahren weiter das Herstellen einer Fluidverbindung zwischen dem arteriellen Leitungsabschnitt des extrakorporalen Blutkreislaufs und einer Quelle mit Zitratlösung.

In erfindungsgemäßen, beispielhaften Ausführungsformen der Steuer- oder Regelungseinrichtung umfasst das Verfahren weiter das Befüllen des extrakorporalen Blutkreislaufs oder vorbestimmter Abschnitte hiervon mittels Blut oder eines Fluids, welches Blut aufweist oder hieraus besteht. Dieses Befüllen dient noch immer dem Vorbereiten des extrakorporalen Blutkreislaufs, nicht einer Blutbehandlung des Patienten. Es kann dem o. g. Primen zeitlich nachgelagert sein. Der extrakorporale Blutkreislauf kann dabei vom Patienten getrennt sein oder nach Befüllen mit Blut erneut von diesem getrennt werden. Seine Enden können beispielsweise kurzgeschlossen sein.

In bestimmten Ausführungsformen umfasst das Verfahren weiter das Zuführen von Zitratlösung zum Blut z. B. mittels einer Heparinpumpe. Das Zuführen kann vorzugsweise in einen Abschnitt des arteriellen Leitungsabschnitts erfolgen, welcher nahe dem Patientenkonnektor oder nahe der Stelle, an welcher dem Patienten Blut entnommen wird, angeordnet ist. Auf diese Weise wird das Blut möglichst unmittelbar nach Entnahme aus dem Patientengefäß antikoaguliert.

In bestimmten Ausführungsformen umfasst das Verfahren weiter das Fördern des vorgenannten Fluids, beispielsweise Blut, mit welchem der extrakorporale Blutkreislauf nun befüllt ist, innerhalb des extrakorporalen Blutkreislaufs mittels einer Blutpumpe unter oder mittels einer ersten Fördergeschwindigkeit. Er umfasst ferner das gleichzeitige Fördern von Zitrat aus der Quelle mit Zitratlösung in den extrakorporalen Blutkreislauf hinein mittels einer zweiten Fördervorrichtung mittels oder unter einer zweiten Fördergeschwindigkeit. Die zweite Fördergeschwindigkeit weist dabei ein eingestelltes und/oder vorbestimmtes Verhältnis zur ersten Fördergeschwindigkeit auf. Optional wird die zweite Fördergeschwindigkeit bei Bedarf auf dieses Verhältnis nachgeregelt.

Beispielsweise kann die erste Fördergeschwindigkeit 100 ml/min betragen; die zweite Fördergeschwindigkeit kann bei Verabreichung von Zitrat als 4%-ige Trinatriumzitratlösung (136 mmol/L) 176 ml/h betragen.

In einigen Ausführungsformen laufen die Blutpumpe und die zweite Fördervorrichtung gleichzeitig an.

In allen erfindungsgemäßen Ausführungsformen ist ein Dialysierflüssigkeitsfluss während des offenbarten, nicht-erfindungsgemäßen Verfahrens angehalten bzw. Null.

In einigen Ausführungsformen wird das vorgenannte Fluid, mit welchem der extrakorporale Blutkreislauf nun befüllt ist, innerhalb des extrakorporalen Blutkreislaufs wenigstens so lange gefördert, bis das Fluid an allen Abschnitten des extrakorporalen Blutkreislaufs vorliegt oder bis es den extrakorporalen Blutkreislauf vollständig ausfüllt.

In manchen Ausführungsformen wird das vorgenannte Fluid, mit welchem der extrakorporale Blutkreislauf nun befüllt ist, innerhalb des extrakorporalen Blutkreislaufs vorzugsweise mittels der Blutpumpe, wenigstens über eine Zeitdauer gefördert, bis sich eine Schicht an Proteinen in oder an einer oder mehreren Oberflächen des extrakorporalen Blutkreislauf ablagert oder abgelagert hat.

In bestimmten Ausführungsformen wird das vorgenannte Fluid, mit welchem der extrakorporale Blutkreislauf nun befüllt ist, innerhalb des extrakorporalen Blutkreislaufs, z. B. mittels Blutpumpe, höchstens über eine Zeitdauer gefördert, welche eine Kalziuminfusion zur Vermeidung einer Hypokalziämie nicht erforderlich macht. Diese Zeitdauer beträgt in gewissen erfindungsgemäßen Ausführungsformen zwischen 10 und 15 min, vorzugsweise zwischen 12 und 13 min, besonders bevorzugt 12,5 min.

In einigen Ausführungsformen wird das Fluid, mit welchem der extrakorporale Blutkreislauf befüllt ist, maximal 15 min gefördert und/oder minimal 3 min, vorzugweise aber zumindest, bis der extrakorporale Blutkreislauf vollständig befüllt ist.

In manchen Ausführungsformen umfasst das Bestimmen einer Höchstmenge an Zitrat, welche verwendet/zugeführt wird, wenigstens die Berücksichtigung von individuellen Daten des Patienten, insbesondere seines extrazellulären Körperwassers. Solche Daten können beispielsweise mittels BCM (kurz für: Body Composition Monitor) ermittelt oder abgeschätzt werden. Hierzu eignet sich u. a. eine Bioimpedanzmessvorrichtung, etwa aus dem Hause der Anmelderin, oder von Xitron Technologies, vermarktet unter der Marke Hydra™ wie in der WO 92/19153 beschrieben.

In manchen Ausführungsformen beträgt die Menge an Zitat, welche der Priminglösung und/oder dem Blut zu Beginn oder bis zum Beginn der Blutbehandlung zugegebenen wird, höchstens 5mmol. Dieser Wert kann einer worst-case-Betrachtung entsprechen.

Das typische Zitratverteilungsvolumen beträgt 25-30% des Körpergewichts. Bei einem kleinen Patienten mit 30 kg Körpergewicht beträgt das Verteilungsvolumen damit 7,5 Liter. Der Anstieg der Zitratkonzentration im Blut durch die infundierten 5 mmol Zitrat beträgt dann 0,67 mmol/l, was akzeptiert wird.

In allen erfindungsgemäßen Ausführungsformen ist die Steuer- oder Regelungseinrichtung ferner programmiert, um nach dem Primen gemäß einer wie oben beschriebenen Ausführungsform im Zusammenwirken mit einer Blutbehandlungsvorrichtung, beispielsweise wie hierin beschrieben, eine Blutbehandlung durchzuführen oder die Blutbehandlung oder die Blutbehandlungsvorrichtung zu steuern oder zu regeln. Dabei wird dem extrakorporalen Blutkreislauf und/oder dem Patienten während der Blutbehandlung kein Heparin oder anderes Antikoagulanz und/oder kein Kalzium, beispielsweise in Form einer zur Beeinflussung der Gerinnung verabreichten Kalziumlösung, beispielsweise mit einer Konzentration von mehr als 5 mmol/L, zugegeben.

In einigen Ausführungsformen wird die Blutpumpe beim sich dem Primen anschließenden Blutbehandlungsverfahren zunächst vergleichsweise langsamer als später und später vergleichsweise schneller als zunächst eingestellt.

Eine Zitratlösung weist in einigen erfindungsgemäßen Ausführungsformen Zitrat auf, wie dies in der US 2006/0037910 A1 und insbesondere in deren Absätzen [0040] bis [0042] beschrieben ist.

In bestimmten erfindungsgemäßen, beispielhaften Ausführungsformen ist die Blutbehandlungsvorrichtung eine Hämodialysevorrichtung, Hämofiltrationsvorrichtung oder Hämodiafiltrationsvorrichtung.

Der extrakorporale Blutkreislauf ist in bestimmten Ausführungsformen der vorliegenden Erfindung ein Schlauchset. In jedem Fall ist der extrakorporale Blutkreislauf vorgesehen zum extrakorporalen Leiten von Blut eines Patienten, beispielsweise bei der Hämodialyse, der Hämofiltration, der Hämodiafiltration oder dergleichen.

In einigen erfindungsgemäßen Ausführungsformen ist der extrakorporale Blutkreislauf zumindest abschnittsweise als integraler und ggf. unlösbarer Bestandteil einer Blutkassette ausgestaltet, in anderen nicht. So kann sich ein frei beweglicher Schlauchabschnitt des extrakorporalen Blutkreislaufs einstückig oder integral auf oder in der Funktionseinrichtung, beispielsweise einer Blutkassette, fortsetzen und umgekehrt.

Eine Blutkassette ist in bestimmten Ausführungsformen der vorliegenden Erfindung eine Einrichtung, welche bei einer Blutbehandlung verwendet wird. Beispiele für Blutkassetten schließen Disposables, Einmal-Blutkassetten ein. Beispielhafte Ausführungsformen einer Blutkassette sind insbesondere in der Anmeldung der Anmelderin mit der Veröffentlichungsnummer DE 10 2009 018 664 A1 mit dem Titel *"Externe Funktionseinrichtung, Blutbehandlungsvorrichtung zum Aufnehmen einer erfindungsgemäßen externen Funktionseinrichtung, sowie Verfahren",* die am 23.04.2009 beim Deutschen Patent- und Markenamt eingereicht wurde, und in der Anmeldung der Anmelderin mit der Veröffentlichungsnummer DE 10 2009 024 468 A1 mit demselben Titel, die am 10.06.2009 beim Deutschen Patent- und Markenamt eingereicht wurde, offenbart. Auf die diesbezüglichen Offenbarungen dieser beiden Anmeldungen wird hiermit jeweils vollinhaltlich Bezug genommen.

Der arterielle Leitungsabschnitt des extrakorporalen Blutkreislaufs ist in bestimmten Ausführungsformen der vorliegenden Erfindung jener Leitungsabschnitt, in welchen das den Patientenkörper zum Zwecke der extrakorporalen Blutbehandlung verlassende Patientenblut einströmt und in welchem es sich vor Eintritt in die Blutbehandlungseinrichtung, z. B. einen Dialysator, befindet.

In bestimmten Ausführungsformen der vorliegenden Erfindung ist oder umfasst der erste Abschnitt des arteriellen Leitungsabschnitts den arteriellen Nadelanschluss zum Patienten, z. B. den arteriellen Nadelanschluss bei einem Double-Needle-Dialyseverfahren.

Der venöse Leitungsabschnitt des extrakorporalen Blutkreislaufs ist in manchen Ausführungsformen der vorliegenden Erfindung jener Leitungsabschnitt, aus welchem das extrakorporal behandelte Patientenblut nach seiner Behandlung in einer Blutbehandlungseinrichtung, beispielsweise einem Dialysator, zum Patientenkörper hin oder in diesen zurückströmt.

In bestimmten Ausführungsformen der vorliegenden Erfindung entspricht die - während der Blutbehandlung übliche - erste Förderrichtung einer Förderrichtung vom arteriellen Zugang (Blutentnahmestelle) des Patienten zu einer Blutbehandlungseinrichtung, beispielsweise einem Blutfilter oder einem Dialysator, und anschließend durch den venösen Leitungsabschnitt des extrakorporalen Blutkreislaufs zum venösen Zugang (Blutrückgabestelle) des Patienten. Alle Einrichtungen, die in der ersten Förderrichtung bezogen auf einen Referenzpunkt des Strömungsweges liegen, liegen stromab hierzu. Solche Einrichtungen, die bezogen zum Referenzpunkt entgegen der ersten Förderrichtung liegen, liegen stromauf hierzu.

Die erfindungsgemäße Steuereinrichtung ist in einigen erfindungsgemäßen Ausführungsformen als Regeleinrichtung ausgestaltet.

Manche oder alle erfindungsgemäßen Ausführungsformen können einen, mehrere oder alle der oben und/oder im Folgenden genannten Vorteile aufweisen.

Ein mittels mancher Ausführungsformen der vorliegenden Erfindung erzielbarer Vorteil besteht darin, dass ein Verfahren sowie entsprechende Vorrichtungen vorgeschlagen werden, mittels welchen ein extrakorporaler Blutkreislauf derart für eine Blutbehandlung vorbereitet wird, dass Letztere ohne Einsatz von Heparin durchgeführt werden kann. Dies bietet angesichts der Tatsache, dass es beispielsweise Dialysepatienten gibt, welche unter einer Heparinunverträglichkeit leiden, erhebliche Vorteile. Es bietet ferner Schutz für solche Patienten, die eine Heparinunverträglichkeit haben, ohne dass ihnen dies bewusst ist.

Die Erfindung kann somit ein Beitrag zur Reduktion der Komplikationen bei heparinfreier Dialyse sein. Es kann insbesondere bei Patienten mit Heparin induzierter Thrombozytopenie (HIT) und bei postoperativen Patienten indiziert sein.

Neben dem Aspekt des Schutzes des Patienten im Falle einer solchen Heparinunverträglichkeit bietet die vorliegende Erfindung einen weiteren Vorteil, welcher darin besteht, dass selbst bei Patienten, welche keine Heparinunverträglichkeit haben, Heparin, ein an sich teurer Wirkstoff, eingespart werden kann.

Vorteilhaft kann ferner sein, dass bei einigen erfindungsgemäßen Ausführungsformen keine über den gesamten Blutbehandlungsverlauf durchzuführende Zitratgabe erforderlich ist. Denn eine solche, andauernde Zitratgabe würde bekanntermaßen erfordern, dass zwingend die physiologische Kalziumkonzentration des behandelten Bluts durch Infusion einer kalziumhaltigen Substitutionslösung wiederhergestellt wird. Hierzu muss allerdings die erforderliche Kalziumkonzentration ermittelt werden. Dies ist aufwendig und mit zusätzlichen Laborkontrollen des Patientenblutes verbunden.

Durch die Beschränkung der Antikoagulanz - ganz oder im Wesentlichen - auf ein kleines Zitratvolumen bei Erstkontakt des Blutes kann also vorteilhaft auf die aufwendige Regelung der Kalzium-Reinfusion verzichtet werden.

Dabei genügt die in der Dialyse übliche Variation der Bikarbonatkonzentration, um den Säure- Basenhaushalt zu kontrollieren. Damit entfallen in der klinischen Routine und vor allem auch ohne speziell auf Zitratantikoagulation geschultes und routiniertes Personal zusätzliche Risiken, wie z. B. Hypo- oder Hyperkalzämie oder starke Verschiebungen des Säure-Basenhaushaltes.

Durch die Vermeidung von durch Heparin induzierten Komplikationen beim Patienten, wie oben ausgeführt, kann der zu ihrer Behebung erforderliche Aufwand und Personaleinsatz entfallen. Auch der bei Komplikationen regelmäßig auftretende Verlust von Blut aufgrund von Gerinnung, welches dem Patienten ggf. durch Blutkonserven zurückgegeben werden muss, kann bei Vermeidung der Komplikationen vorteilhaft verhindert werden.

Im Folgenden wird die vorliegende Erfindung unter Bezugnahme auf die beigefügte Figur rein exemplarisch beschrieben. Es gilt:

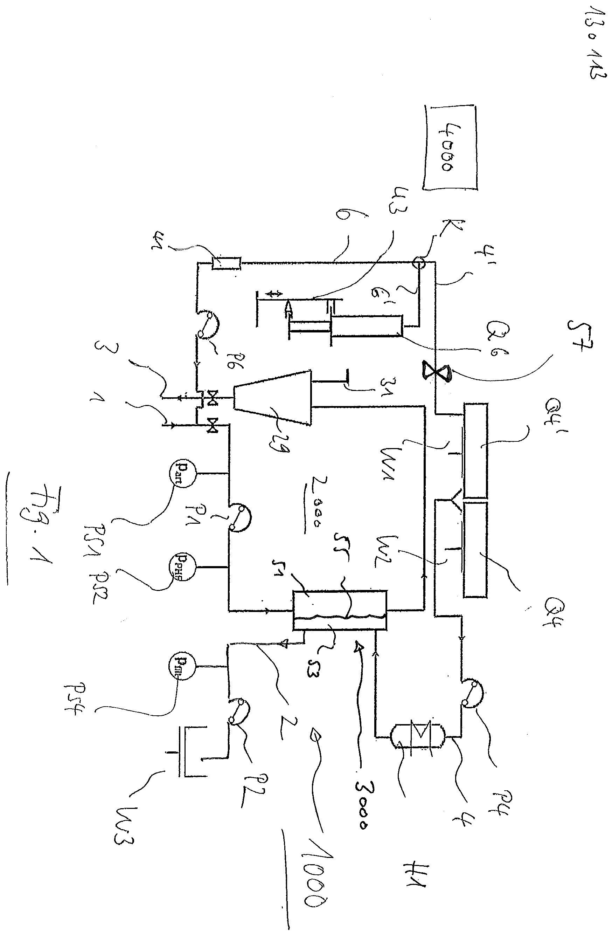

- **Fig.** 1: zeigt in vereinfachter Darstellung eine erfindungsgemäße Blutbehandlungsvorrichtung mit einem extrakorporalen Blutkreislauf in einer beispielhaften Ausführungsform.

**Fig. 1** zeigt in vereinfachter Darstellung eine erfindungsgemäße Blutbehandlungsvorrichtung 1000 mit einem extrakorporalen Blutkreislauf 2000.

Der extrakorporale Blutkreislauf 2000 weist eine Blutbehandlungseinrichtung 3000, hier exemplarisch einen Blutfilter oder Dialysator mit einer Blutkammer 51, einer Dialysierflüssigkeitskammer 53 und einer Membran 55 auf oder ist mit der Blutbehandlungseinrichtung 3000 verbunden. Ein arterieller Leitungsabschnitt 1 des extrakorporalen Blutkreislaufs 2000 führt Blut vom Gefäßsystem des nicht dargestellten Patienten in Richtung zum Blutfilter 3000. Ein venöser Leitungsabschnitt 3 des extrakorporalen Blutkreislaufs 2000 führt Blut vom Blutfilter 3000 in Richtung zum Gefäßsystem des nicht dargestellten Patienten.

Die in Fig. 1 nur durch einige ihrer Einrichtungen repräsentierte Blutbehandlungsvorrichtung 1000, mittels welcher das hierin beschriebene Verfahren abläuft, weist eine Blutpumpe P1 auf. Die Blutpumpe P1 fördert Blut durch Abschnitte des extrakorporalen Blutkreislaufs 2000 und in Richtung zum Blutfilter 3000, wie die kleinen Pfeilspitzen, welche in der Figur allgemein die Strömungsrichtung angeben, zeigen.

Neben der vorgenannten Pumpe P1 weist die in Fig. 1 gezeigte Anordnung ferner rein optional eine Reihe weiterer Pumpen P2, P4 und P6 auf.

Zu den optionalen Pumpen zählt die Pumpe P4. Sie ist vorgesehen, um Dialysierflüssigkeit aus einer entsprechenden Quelle Q4, beispielsweise einem Beutel, heraus und der Dialysierflüssigkeitskammer 53 über eine optional vorhandene Beutelheizung mit einem Beutel H1 mittels einer Dialysierflüssigkeitsleitung 4 zuzuführen.

Die auf diese Weise zugeführte Dialysierflüssigkeit tritt über eine Dialysatleitung 2, optional unterstützt durch eine Pumpe P2, wieder aus und kann verworfen werden.

Die optionale Pumpe P6 fördert ein kombiniertes Fluid aus Priminglösung und Zitratlösung, im Weiteren der Einfachheit halber als Priminglösung bezeichnet, entlang einer Leitung 6, hier als gemeinsame Leitung zu verstehen, in den arteriellen Leitungsabschnitt 1 hinein. Diese Funktion kann anstelle durch die Pumpe P6 auch ausschließlich durch die Blutpumpe P1 erfolgen. Auf die Pumpe P6 kann daher verzichtet werden.

Die Priminglösung entstammt beispielsweise einer ersten Quelle Q4', hier beispielhaft ein Beutel mit Dialysierflüssigkeit (könnte aber auch Substituat, Kochsalzlösung oder eine andere Lösung oder Flüssigkeit sein), und wird zunächst bis zum Kreuzungspunkt K in der ersten Leitung 4' gefördert.

Die Zitratlösung entstammt einer zweiten Quelle Q6, hier einer Spritze mit Zitrat oder Zitratkonzentrat ("Ci-Konzentrat"), und wird zunächst bis zum Kreuzungspunkt K in einer zweiten Leitung 6' gefördert. Die Spritze verfügt über eine eigene Fördereinrichtung, eine hier nicht dargestellte Spritzenpumpe. Für den Fachmann ist ersichtlich, dass es einer solchen Spritzenpumpe jedenfalls dann nicht bedarf, wenn die zweite Quelle Q6 keine Spritze sondern beispielsweise ein Beutel ist. In diesem Fall ist die Fördereinrichtung eine Pumpe und nicht optional.

Ab dem Kreuzungspunkt oder Verbindungspunkt K gehen die erste Leitung 4' und die zweite Leitung 6' in die gemeinsame Leitung 6 über oder münden in diese.

Im vorliegenden Beispiel sind die erste Quelle Q4' und die Quelle Q4 voneinander unabhängige Quellen für ein und dasselbe Fluid, nämlich Dialysierflüssigkeit. Von der Erfindung umfasst ist aber auch, dass Q4 und Q4' ein und dieselbe Quelle oder aber miteinander in Fluidverbindung stehende Quellen sind.

Stromauf der Blutpumpe P1 ist ein optionaler arterieller Sensor PS1 vorgesehen, er misst den arteriellen Druck P_art.

Stromab der Blutpumpe P1, jedoch stromauf des Blutfilters 3000, ist ein weiterer, optionaler Drucksensor PS2 vorgesehen. Er misst den Druck stromauf des Blutfilters 3000 (PHF steht für "prä-Hämofilter").

Ein wiederum weiterer Drucksensor kann als PS4 stromab des Blutfilters 3000, jedoch stromauf der Pumpe P2 in der Dialysatleitung 2 zum Messen eines Filterdrucks des Blutfilters 3000 vorgesehen sein.

Blut, das den Blutfilter 3000 verlässt, durchströmt eine venöse Blutkammer 29, welche eine Entlüftungseinrichtung 31 aufweisen kann.

Im Beispiel der Fig. 1 unterliegen die Quellen Q4 und Q4' sowie das aufgefangene oder verworfene Dialysat optional einer Bilanzierung. Zum Zweck der optionalen Bilanzierung sind drei Waagen W1, W2 und W3 vorgesehen.

Die hier exemplarisch gezeigte Bilanzierung entspricht einer gravimetrischem Bilanzierung. Die vorliegende Erfindung umfasst aber auch jede andere Bilanzierung, beispielsweise mittels Bilanzkammern.

Die in Fig. 1 gezeigte exemplarische Anordnung weist optional eine erste Überwachungsvorrichtung 41 auf. Sie ist ausgestaltet zum Überwachen der korrekten Funktion und/oder zum Ermitteln einer Förderleistung oder eines Fördervolumens der Pumpe P6.

Die in Fig. 1 gezeigte exemplarische Anordnung weist optional ferner eine zweite Überwachungsvorrichtung 43 auf. Sie ist ausgestaltet zum Überwachen der korrekten Funktion und/oder zum Ermitteln einer Förderleistung oder eines Fördervolumens der zweiten Fördereinrichtung, hier der Spritzenpumpe, der zweiten Quelle Q6. Die zweite Überwachungsvorrichtung 43 ist, rein optional, erkennbar als Wegesensor ausgestaltet. Sowohl die erste als auch die zweite Überwachungsvorrichtung 41, 43 können als Tropfenzähler ausgestaltet sein. Sowohl die erste als auch die zweite Überwachungsvorrichtung 41, 43 können mit einer in den Figuren nicht dargestellten Vergleichseinrichtung verbunden sein.

Eine erfindungsgemäße Steuer- oder Regeleinrichtung 4000 ist in Fig. 1 angedeutet. Sie steht in Signalverbindung mit allen relevanten Einrichtungen, jedenfalls mit der Blutpumpe P1 und vorzugsweise auch mit der Pumpe P6 oder der Spritzenpumpe.

Folgende Merkmale können, obgleich in den Figuren nicht gezeigt, in jeder erfindungsgemäßen Ausführungsform wiederum rein optional und in beliebiger Kombination vorgesehen sein:
Der arterielle Leitungsabschnitt 1 kann eine arterielle Klemme aufweisen.

Der arterielle Leitungsabschnitt 1 kann ein arterielles Septum, optional in Gestalt einer Zugabeeinrichtung, aufweisen.

Der venöse Leitungsabschnitt 3 kann einen venösen Luftblasendetektor/optischen Sensor aufweisen.

Der venöse Leitungsabschnitt 3 kann eine venöse Klemme aufweisen.

Der arterielle Leitungsabschnitt 3 kann einen arteriellen Luftblasendetektor/optischen Sensor aufweisen.

Im Folgenden wird eine mögliche, mittels der Anordnung der Fig. 1 durchführbare Ausführungsform der Erfindung beschrieben, bei welcher die Interaktion zwischen Blut und extrakorporalem Kreislauf während der initialen Phase wesentlich gedämpft ist.

Hierzu wird sowohl die Priminglösung als auch das initiale Blutvolumen derart mit Zitrat antikoaguliert, dass die Gerinnung effektiv gehemmt ist. Andererseits wird die Gesamtmenge an infundiertem Zitrat so bemessen, dass eine Infusion von Kalzium zum Vermeiden einer akuten Hypokalziämie nicht erforderlich ist und keine ungewollte Verschiebung des Säure-Basenhaushaltes erfolgt.

Beim Primen mittels Priminglösung wird in einem ersten Schritt Zitrat eingesetzt, um in der sich an das Primen anschließenden, initialen Blutbehandlungsphase, die mit einem vergleichsweise geringen Blutfluss läuft, die Diffusion von Kalzium aus dem Dialysat in das Blutkompartiment zu verhindern. Die einzusetzende Zitratkonzentration ist damit abhängig von der Kalziumkonzentration in der Priminglösung. Die resultierende Kalziumkonzentration in der Priminglösung liegt vorzugsweise unter 0,5 mmol/L.

In einem zweiten Schritt wird über einen begrenzten Zeitraum mit Zitrat antikoaguliertes Blut mit einem möglichst geringen Blutfluss, z. B. 100 ml/min, in den extrakorporalen Kreislauf gepumpt. So soll eine Belegung des Blutfilters 3000 mit körpereigenen Proteinen erreicht werden. Die in dieser Phase zu erreichende Zitratkonzentration im Blut kann dann vorzugsweise 4 mmol/l betragen. Als Zitratlösung kann z. B. eine 4%ige Trinatriumzitratlösung (136 mmol/1) verwendet werden. Bei einem Blutfluss von 100 ml/min würde das einen Zitratfluss von 176 ml/h bedeuten.

Das Blut sollte zumindest so lange antikoaguliert werden, bis der extrakorporale Blutkreislauf einmal gefüllt wurde, d. h. bis der Blutalarm ausgelöst wurde, also an einem Sensor des Blutkreislaufs in Letzterem Blut erkannt wurde, eventuell noch einige Minuten länger.

Ist die Inkubation des extrakorporalen Kreislaufs mit Zitratantikoaguliertem Blut abgeschlossen, wird der Rest der Behandlung ohne Antikoagulanz oder mit einer verminderten Menge Antikoagulanz weitergeführt und mit höherem Blutfluss durchgeführt. Möglich ist auch eine einmalige Bolusgabe von Heparin an den Patienten vor Behandlungsbeginn. Insgesamt wird aber eine Verminderung der Heparinmenge erreicht.

Das Verfahren kann somit grob in zwei zeitlich aufeinander folgende Phasen, Phase 1 und Phase 2, unterteilt werden. In Phase 1 wird der extrakorporale Kreislauf mit Priminglösung befüllt. Ist dies erfolgt, so ist er mit einer Lösung gefüllt, die durch Zugabe von Zitrat < 0,5 mmol/L ionisiertes Kalzium enthält, möglichst einen pH-Wert von über 7,3 hat (um Kontaktphasenaktivierung zu vermeiden), und die sonst übliche physiologische Zusammensetzung besitzt.

Des Weiteren ist es vorteilhaft, dass der Inhalt der Dialysierflüssigkeitskammer 53 des Blutfilters 3000 die gleiche Lösungszusammensetzung besitzt, wie der übrige Blutkreislauf 2000. Dies soll verhindern, dass Kalzium aus der Dialysierflüssigkeit in den Blutkreislauf 3000 diffundiert und damit die Konzentration des ionisierten Kalziums erhöht.

In Phase 2 wird der extrakorporale Blutkreislauf 2000 mit Blut befüllt. Eine konzentrierte Zitratlösung wird mittels einer Dosiervorrichtung (z. B. der Heparinpumpe) dem arteriellen Strang des extrakorporalen Blutkreislaufs 2000 zugeführt. Idealerweise geschieht dies nahe am Konnektor der arteriellen Nadel, um das Blut möglichst unmittelbar nach Entnahme aus dem Körper zu antikoagulieren.

Nachdem die arterielle Nadel mit dem arteriellen Leitungsabschnitt 1 verbunden ist, wird exemplarisch folgende Befüllroutine automatisch oder manuell gestartet: Die Blutpumpe P1 und die Zitratinfusionspumpe laufen gleichzeitig an. Als Zitratinfusionspumpe kann die Pumpe P6 verstanden werden, bei geschlossener Klemme 57. Dabei steuert die Blutpumpe die Geschwindigkeit der Zitratinfusionspumpe über ein einstellbares Übersetzungsverhältnis. Damit wird erreicht, dass die ionisierte Kalziumkonzentration im Blut auf das gewünschte niedrige Niveau für die vollständige Antikoagulation gefahren wird. Die Geschwindigkeit der Blutpumpe P1 wiederum wird dadurch bestimmt, dass einerseits die (einstellbare) Zeit, in der das Blut über die körperfremden Flächen oder Oberflächen des extrakorporalen Blutkreislaufs 2000 fließt, ausreichend lang ist, um darauf eine Sekundärschicht aus Proteinen aufzubauen, und andererseits die einstellbare Zeit kurz genug ist, um keine Kalziuminfusion zur Vermeidung einer Hypokalziämie im Patienten erforderlich zu machen und eine relevante Hypernatriämie oder Alkalose zu vermeiden.

Der Dialysierflüssigkeitsfluss ist während dieses Prozesses gestoppt.

Die Menge an Zitrat, die dem Patienten unbedenklich zugeführt werden kann, sollte aus den Patienten kennzeichnenden Parametern (automatisch oder durch manuelle Eingabe) ermittelt werden. Hierzu ist idealerweise die Information über das Volumen seines extrazellulären Körperwassers zu verwenden, z. B. aus BCM Daten. In der Literatur befinden sich hierzu Referenzen für die verabreichbare Zitratmenge.

Für die Berechnung der Zeit für die Infusion sind einer oder mehrere der folgenden Faktoren zu berücksichtigen:
- das extrakorporale Blutvolumen
- die Entscheidung, ob synchron (d. h. Arterie und Vene gleichzeitig), oder mit Aderlass (d. h. die Priminglösung wird bis zum Erkennen von Blut im venösen Schlauchsystem verworfen) angefahren wird
- die minimale Blutpumpengeschwindigkeit
- die minimale Zeit, um die initiale Proteinadsorption abzuschließen

### Bezugszeichenliste

- 1000: Blutbehandlungsvorrichtung
- 2000: extrakorporaler Blutkreislauf
- 3000: Blutbehandlungseinrichtung, Blutfilter oder Dialysator
- 4000: Steuer- oder Regeleinrichtung
- 1: arterieller Leitungsabschnitt
- 2: Dialysatleitung
- 3: venöser Leitungsabschnitt
- 4: Dialysierflüssigkeitsleitung
- 4': erste Leitung, führt aus erster Quelle heraus
- 6: gemeinsame Leitung
- 6': zweite Leitung, führt aus zweiter Quelle heraus
- H1: Beutelheizung mit Beutel

- P1: Blutpumpe
- P2, P4, P6: optionale Pumpen
- PS1: arterieller Sensor, misst den arteriellen Druck P_art
- PS2: optionaler Drucksensor

- PS4: Drucksensor zum Messen eines Filterdrucks

- Q4: Quelle mit Dialysierflüssigkeit
- Q4': erste Quelle
- Q6: zweite Quelle

- 29: venöse Blutkammer
- 31: Entlüftungseinrichtung
- K: Kreuzungspunkt oder Verbindungspunkt der ersten Leitung mit der zweiten Leitung
- W1, W2, W3: Waagen
- 41: erste Überwachungsvorrichtung
- 43: zweite Überwachungsvorrichtung
- 51: Blutkammer
- 53: Dialysierflüssigkeitskammer
- 55: Membran
- 57: geschlossene Klemme

## Patentansprüche

1. Steuer- oder Regelungseinrichtung (4000) programmiert, um im Zusammenwirken mit einer Blutbehandlungsvorrichtung (1000) eine Blutbehandlung durchzuführen, und programmiert zur Durchführung eines Verfahrens zum Vorbereiten eines extrakorporalen Blutkreislaufs (2000) für seine Verwendung in einer mittels der Blutbehandlungsvorrichtung (1000) und mittels einer Blutbehandlungseinrichtung (3000), welche eine Blutkammer (51) und eine mittels Membran (55) hiervon abgeteilte Dialysierflüssigkeitskammer (53) aufweist, durchgeführten Blutbehandlung eines Patienten, wobei das Verfahren zum Vorbereiten des extrakorporalen Blutkreislaufs bei angehaltenem oder gestopptem Dialysierflüssigkeitsfluss oder wenn der Dialysierflüssigkeitsfluss Null ist, durchgeführt wird, und wobei das Verfahren die folgenden Schritte aufweist:
- Befüllen des extrakorporalen Blutkreislaufs (2000) mittels einer Priminglösung, wobei die Priminglösung Zitrat aufweist, oder Befüllen des extrakorporalen Blutkreislaufs (2000) mittels einer Priminglösung und mittels einer Zitratlösung;
- Befüllen des extrakorporalen Blutkreislaufs (2000) mittels eines Fluids, welches Blut aufweist oder hieraus besteht.

2. Steuer- oder Regelungseinrichtung (4000) nach Anspruch 1, wobei die Priminglösung, mit welcher der extrakorporale Blutkreislauf (2000) befüllt wird, maximal 0,5 mmol/L Kalzium aufweist.

3. Steuer- oder Regelungseinrichtung (4000) nach Anspruch 1 oder 2, wobei das Verfahren weiter das Zuführen von Zitratlösung zum Blut umfasst.

4. Steuer- oder Regelungseinrichtung (4000) nach einem der vorangegangenen Ansprüche, wobei die Priminglösung, mit welcher der extrakorporale Blutkreislauf (2000) befüllt wird, einen pH-Wert von wenigstens 7,3 aufweist.

5. Steuer- oder Regelungseinrichtung (4000) nach einem der vorangegangenen Ansprüche, wobei das Verfahren weiter umfasst:
- Herstellen einer Fluidverbindung zwischen dem arteriellen Leitungsabschnitt (1) des extrakorporalen Blutkreislaufs (2000) und einer Quelle (Q6) mit Zitratlösung.

6. Steuer- oder Regelungseinrichtung (4000) nach einem der vorangegangenen Ansprüche, wobei das Verfahren weiter umfasst:
- Fördern des Fluids, mit welchem der extrakorporale Blutkreislauf (2000) befüllt ist, innerhalb des extrakorporalen Blutkreislaufs (2000) mittels einer Blutpumpe (P1) mittels einer ersten Fördergeschwindigkeit; und
- gleichzeitiges Fördern von Zitrat aus einer zweiten Quelle (Q6) mit Zitratlösung in den extrakorporalen Blutkreislauf (2000) hinein mittels einer zweiten Fördervorrichtung (P6) mittels einer zweiten Fördergeschwindigkeit;
wobei die zweite Fördergeschwindigkeit in einem eingestellten und/oder vorbestimmten Verhältnis zur ersten Fördergeschwindigkeit eingestellt wird.

7. Steuer- oder Regelungseinrichtung (4000) nach einem der vorangegangenen Ansprüche, wobei das Fluid, mit welchem der extrakorporale Blutkreislauf (2000) befüllt ist, innerhalb des extrakorporalen Blutkreislaufs (2000) mittels einer Blutpumpe (P1) wenigstens über eine Zeitdauer gefördert wird, bis sich eine Schicht an Proteinen an einer oder mehreren Oberflächen des extrakorporalen Blutkreislaufs (2000) ablagert.

8. Steuer- oder Regelungseinrichtung (4000) nach einem der vorangegangenen Ansprüche, wobei das Fluid, mit welchem der extrakorporale Blutkreislauf (2000) befüllt ist, innerhalb des extrakorporalen Blutkreislaufs (2000) mittels einer Blutpumpe (P1) höchstens über eine Zeitdauer gefördert wird, welche eine Kalziuminfusion zur Vermeidung einer Hypokalziämie nicht erforderlich macht.

9. Steuer- oder Regelungseinrichtung (4000) nach einem der vorangegangenen Ansprüche, wobei das Verfahren weiter umfasst:
- Bestimmen einer Höchstmenge an Zitrat, welche verwendet/zugeführt wird, unter Berücksichtigung von individuellen Daten des Patienten, insbesondere seines extrazellulären Körperwassers.

10. Steuer- oder Regelungseinrichtung nach Anspruch 9, ferner programmiert, um während der Blutbehandlung kein Heparin und/oder kein Kalzium dem im extrakorporalen Blutkreislauf (2000) geführten Blut zuzugeben.

11. Blutbehandlungsvorrichtung (1000), aufweisend eine Steuer- oder Regelvorrichtung (4000) gemäß einem der Ansprüche 1 bis 10.

12. Blutbehandlungsvorrichtung nach Anspruch 11, ausgestaltet als Hämodialysevorrichtung, Hämofiltrationsvorrichtung oder Hämodiafiltrationsvorrichtung.

## Claims

1. A control or regulating device (4000) programmed to perform a blood treatment in cooperation with a blood treatment apparatus (1000) and programmed to perform a method for preparing an extracorporeal blood circuit (2000) for its use in a blood treatment on a patient, performed by means of the blood treatment apparatus (1000) and by means of a blood treatment device (3000), which comprises a blood chamber (51) and a dialysis liquid chamber (53) separated therefrom by means of a membrane (55),
wherein the method for preparing the extracorporeal blood circuit is performed when the dialysis liquid flow is halted or stopped or when the dialysis liquid flow is zero,
and wherein the method comprises the following steps:
- filling the extracorporeal blood circuit (2000) by means of a priming solution, the priming solution containing citrate, or filling the extracorporeal blood circuit (2000) by means of a priming solution and by means of a citrate solution;
- filling the extracorporeal blood circuit (2000) by means of a fluid containing or consisting of blood.

2. The control or regulating device (4000) according to claim 1, wherein the priming solution, with which the extracorporeal blood circuit (2000) is filled, contains a maximum of 0.5 mmol/L calcium.

3. The control or regulating device (4000) according to claim 1 or 2, wherein the method further comprises supplying citrate solution to the blood.

4. The control or regulating device (4000) according to anyone of the preceding claims, wherein the priming solution, with which the extracorporeal blood circuit (2000) is filled, has a pH value of at least 7.3.

5. The control or regulating device (4000) according to anyone of the preceding claims, wherein the method further comprises :
- establishing a fluid connection between the arterial line section (1) of the extracorporeal blood circuit (2000) and a source (Q6) containing citrate solution.

6. The control or regulating device (4000) according to anyone of the preceding claims, wherein the method further comprises :
- conveying the fluid, with which the extracorporeal blood circuit (2000) is filled, within the extracorporeal blood circuit (2000) by means of a blood pump (P1) at a first conveying speed; and
- simultaneously conveying citrate from a second source (Q6) containing citrate solution into the extracorporeal blood circuit (2000) by means of a second conveying apparatus (P6) at a second conveying speed;
wherein the second conveying speed is set in a set and/or predetermined ratio to the first conveying speed.

7. The control or regulating device (4000) according to anyone of the preceding claims, wherein the fluid, with which the extracorporeal blood circuit (2000) is filled, is conveyed within the extracorporeal blood circuit (2000) by means of a blood pump (P1) at least over a period of time until a layer of proteins is deposited on one or more surfaces of the extracorporeal blood circuit (2000).

8. The control or regulating device (4000) according to anyone of the preceding claims, wherein the fluid, with which the extracorporeal blood circuit (2000) is filled, is conveyed within the extracorporeal blood circuit (2000) by means of a blood pump (P1) at most over a period of time which does not necessitate a calcium infusion in order to avoid hypocalcaemia.

9. The control or regulating device (4000) according to anyone of the preceding claims, wherein the method further comprises :
- determining a maximum amount of citrate to be used/added, taking into account individual data of the patient, in particular his extracellular body water.

10. The control or regulating device (4000) according to claim 9, further programmed not to add heparin and/or calcium to the blood circulating in the extracorporeal blood circuit (2000) during the blood treatment.

11. A blood treatment apparatus (1000) comprising a control or regulating device (4000) according to anyone of claims 1 to 10.

12. The blood treatment apparatus (1000) according to claim 11, designed as a hemodialysis apparatus, a hemofiltration apparatus or a hemodiafiltration apparatus.

## Revendications

1. Un dispositif de commande ou de régulation (4000) programmé pour effectuer un traitement du sang en coopération avec un appareil de traitement du sang (1000) et programmé pour effectuer un procédé de préparation d'un circuit sanguin extracorporel (2000) pour son utilisation dans un traitement du sang d'un patient, effectué au moyen de l'appareil de traitement du sang (1000) et au moyen d'un dispositif de traitement du sang (3000), qui comprend une chambre à sang (51) ainsi qu'une chambre à liquide de dialyse (53) séparée de celle-ci par une membrane (55),
où le procédé de préparation du circuit sanguin extracorporel est effectué lorsque le flux de liquide de dialyse est interrompu ou arrêté, ou lorsque le flux de liquide de dialyse est nul, et où le procédé comprend les étapes suivantes:
- le remplissage du circuit sanguin extracorporel (2000) au moyen d'une solution d'amorçage, la solution d'amorçage contenant du citrate, ou le remplissage du circuit sanguin extracorporel (2000) au moyen d'une solution d'amorçage ainsi que d'une solution de citrate;
- le remplissage du circuit sanguin extracorporel (2000) au moyen d'un fluide contenant ou constitué de sang.

2. Le dispositif de commande ou de régulation (4000) selon la première revendication, où la solution d'amorçage, dont le circuit sanguin extracorporel (2000) est rempli, contient au maximum 0,5 mmol/L de calcium.

3. Le dispositif de commande ou de régulation (4000) selon la revendication 1 ou 2, où le procédé comprend en outre l'ajout d'une solution de citrate dans le sang.

4. Le dispositif de commande ou de régulation (4000) selon l'une quelconque des revendications précédentes, où la solution d'amorçage, dont le circuit sanguin extracorporel (2000) est rempli, a un pH d'au moins 7,3.

5. Le dispositif de commande ou de régulation (4000) selon l'une quelconque des revendications précédentes, où le procédé comprend en outre:
- l'établissement d'une liaison fluidique entre la section de la ligne artérielle (1) du circuit sanguin extracorporel (2000) et une source (Q6) contenant une solution de citrate.

6. Le dispositif de commande ou de régulation (4000) selon l'une quelconque des revendications précédentes, où le procédé comprend en outre:
- l'acheminement du fluide, dont le circuit sanguin extracorporel (2000) est rempli, dans le circuit sanguin extracorporel (2000) au moyen d'une pompe à sang (P1) à une première vitesse d'acheminement; et
- l'acheminement simultané de citrate à partir d'une seconde source (Q6) contenant une solution de citrate dans le circuit sanguin extracorporel (2000) au moyen d'un second appareil d'acheminement (P6) à une seconde vitesse d'acheminement;
où la seconde vitesse d'acheminement est fixée dans un rapport fixé et/ou prédéterminé par rapport à la première vitesse d'acheminement.

7. Le dispositif de commande ou de régulation (4000) selon l'une quelconque des revendications précédentes, où le fluide, dont le circuit sanguin extracorporel (2000) est rempli, est acheminé dans le circuit sanguin extracorporel (2000) au moyen d'une pompe à sang (P1) au moins sur une période de temps durant laquelle une couche de protéines se dépose sur une ou plusieurs surfaces du circuit sanguin extracorporel (2000).

8. Le dispositif de commande ou de régulation (4000) selon l'une quelconque des revendications précédentes, où le fluide, dont le circuit sanguin extracorporel (2000) est rempli, est acheminé dans le circuit sanguin extracorporel (2000) au moyen d'une pompe à sang (P1) au plus sur une période de temps qui ne rend pas nécessaire une perfusion de calcium pour éviter une hypocalcémie.

9. Le dispositif de commande ou de régulation (4000) selon l'une quelconque des revendications précédentes, où le procédé comprend en outre:
- la détermination d'une quantité maximale de citrate à utiliser/ajouter, tenant compte des données individuelles du patient, en particulier de son eau corporelle extracellulaire.

10. Le dispositif de commande ou de régulation (4000) selon la revendication 9, programmé en outre pour ne pas ajouter d'héparine et/ou de calcium dans le sang circulant dans le circuit sanguin extracorporel (2000) pendant le traitement du sang.

11. Un appareil de traitement du sang (1000) comprenant un dispositif de commande ou de régulation (4000) selon l'une quelconque des revendications 1 à 10.

12. L'appareil de traitement du sang (1000) selon la revendication 11, conçu comme un appareil d'hémodialyse, un appareil d'hémofiltration ou un appareil d'hémodiafiltration.
